# EUROPÄISCHE PATENTSCHRIFT

(11) **EP 2 320 754 B1**
(45) Veröffentlichungstag und Bekanntmachung des Hinweises auf die Patenterteilung: **01.01.2020**
(21) Anmeldenummer: 09748696.3
(22) Anmeldetag: 22.07.2009
(51) Int. Cl.: A61K 9/107, A61K 9/00, A61K 8/06, A61K 8/98, A61K 35/64, A61Q 19/08

(54) **MIKROEMULSION**
MICROEMULSION
MICROÉMULSION

(30) Priorität: 26.07.2008 DE 102008034944
(43) Veröffentlichungstag der Anmeldung: 18.05.2011
(73) Patentinhaber: Teslenko, Alexander, 58095 Hagen (DE); Arivine Pharma AG, 8008 Zürich (CH)
(72) Erfinder: TESLENKO, Alexander, 58095 Hagen (DE)
(74) Vertreter: Schöneborn, Holger
(86) Internationale Anmeldenummer: PCT/EP2009/005318
(87) Internationale Veröffentlichungsnummer: WO 2010/012408

(56) Entgegenhaltungen:
- EP-A2- 0 216 557
- WO-A1-89/06955
- WO-A1-99/55349
- WO-A1-2007/137881
- WO-A1-2009/067734
- WO-A2-2006/074177
- GB-A- 2 113 523
- US-A- 5 688 761

## Beschreibung

Honig ist ein Naturprodukt und besteht aus etwa 200 verschiedenen Inhaltsstoffen. Die Zusammensetzung kann je nach Honigsorte sehr unterschiedlich sein. Die mengenmäßig wichtigsten Inhaltsstoffe sind Fructose (27 bis 40 %), Glucose (22 bis 41 %) und Wasser (ca. 18 %). Weitere typische Inhaltsstoffe sind andere Zuckerarten, Pollen, Mineralstoffe, Proteine, Enzyme, Aminosäuren, Vitamine, Farb- und Aromastoffe. Honig kann flüssig oder auch fest (kristallisiert) sein. Dies hängt hauptsächlich von dem Verhältnis der beiden Einfachzucker Frucht- und Traubenzucker zueinander ab, aber auch davon, wie der Honig weiterverarbeitet und gelagert wird.

Honig wird seit der Steinzeit von Menschen genutzt und war lange Zeit das einzige Süßungsmittel. Infolge der Entwicklung von Verfahren zur Herstellung von billigem Haushaltszucker (reine Saccharose) aus Zuckerrüben und Zuckerrohr ist Honig in dieser Hinsicht weitgehend verdrängt worden. Trotzdem wird Honig als Nahrungsmittel, zum Beispiel als süßer Brotaufstrich oder als Alternative zum industriell hergestellten Haushaltszucker, weiterhin geschätzt. Heutzutage erlebt Honig durch seine positiven gesundheitsunterstützenden Wirkungen eine Renaissance. Viele der in Honig enthaltenen Stoffe haben nicht nur ernährungsrelevante Bedeutung, sondern auch positive gesundheitliche Wirkungen auf Menschen und Tiere.

Neben anderen Bienenprodukten wurde Honig schon vor Jahrtausenden auch in der Naturheilkunde als Heilmittel eingesetzt. In neuerer Zeit wird ein speziell vorverarbeiteter, keimfrei gemachter Honig (sog. medizinischer Honig) auch zur Wundversorgung eingesetzt. In vielen Ländern befinden sich eine Reihe von Honig-Präparaten auf dem Markt, die als Medizinprodukte für die Wundheilung in Form von Gelen, Wundauflagen oder Pads zugelassen sind.

Naturbelassener Honig eignet sich jedoch nicht zur Wundbehandlung. Zwar können sich Keime im Honig nicht vermehren, aber es kann nicht ganz ausgeschlossen werden, dass sie als Verunreinigung im Honig vorhanden sind. Der für medizinische Zwecke angewandte Honig wird deshalb vor der Anwendung mit Hilfe von Gammastrahlen sterilisiert. Im Gegensatz zur thermischen Sterilisation werden hierbei die an der Heilwirkung maßgeblich beteiligten Enzyme nicht zerstört.

Auf Grund der zunehmenden Zahl antibiotikaresistenter Keime sind die bakteriziden und bakteriostatischen Wirkungen von Honig sehr interessant vor allem in der Medizin. Sogar 10-40 %iger Honig tötet antibiotikaresistente gram-negative Bakterien nach zwei Tagen Applikation ab (P. H. Kwakmann et al., Clin Infect. Dis. 46 (11), 1677-82 (2008); P. J. Taormina et al., 2001, Int. J. Food Microbiol. 69 (3), 217-25 (2001)).

Als wichtigster antibakterieller Wirkstoff des Honigs gilt heute Wasserstoffperoxid (H₂O₂). Wasserstoffperoxid und Gluconsäure entstehen bei der Oxidation von Wasser und Glucose mit Hilfe von Glucoseoxidase. Als Gegenspieler der Glucoseoxidase fungiert die Katalase. Die Bildung von H₂O₂ wird außerdem durch Wärme und Licht beeinflusst. Diese schädigen die Glucoseoxidase und bremsen somit die Produktion von H₂O₂.

Daneben werden in neuerer Zeit noch weitere Inhaltsstoffe (sog. Inhibine) mit bakteriziden, positiven Wirkungen erforscht, die unter anderem Methicillinresistente Staphylokokken und Vancomycin-resistente Enterokokken abtöten. In Honig vorhandene nicht-peroxide Stoffe (Inhibine) stammen vorwiegend von Pflanzen. In unterschiedlichem Maße zeigen vier Gruppen von Stoffen des Honigs ihre bakteriostatischen Wirkungen: Sie sind flüchtiger, neutraler, basischer und säuerlicher Natur. Zu den Inhibinen ordnet man eine Reihe von pflanzlichen Stoffen mit antibakterieller Aktivität: Terpene, Benzylalkohol, Pinocembrin, Methylsyringate, Hydroxybenzolsäure u.a.

Die sehr starke antibiotische Wirkung von Manuka-Honig schreibt man dem Methylglyoxal zu, das in ungewöhnlich hoher Konzentration (oft 1000fach) vorhanden ist.

Nur wenige heilende Wirkungen von Honig wurden in vitro, in vivo und in klinischen Studien ausführlich untersucht. Gut belegt allerdings sind die heilenden Wirkungen von Honig bei topischen Anwendungen.

Honig wirkt leicht entzündungshemmend, so dass Schwellungen, erhöhte Temperatur und lokaler Schmerz zurückgehen. Er fördert das Wachstum von Fibroblasten, wodurch die Wunde gleichmäßiger heilt und es zu weniger Narbenbildung kommt (WO 2007/009185).

Honig wird als Wundauflage benutzt, da er leicht antiseptisch wirkt und zudem in Wunden vorhandenes totes Gewebe abbaut. Mittlerweile sind drei antimikrobielle Wirkmechanismen bekannt, nämlich die sehr hohe Osmolarität, Wasserstoffperoxid und Inhibine.

Honig wird in verschiedenen Darreichungsformen in der topischen Wundversorgung angewendet. Eine Zusammensetzung in Form einer Salbe aus Honig, Olivenöl und Bienenwachs hat sich gut bei topischen Behandlungen von Hämorrhoiden und Analfissuren bewährt. Auch mit Honig imprägnierte poröse und nicht poröse Wundauflagen sind bekannt (WO 2007/137881).

Aus der WO 99/55349 A1 wird die Verwendung eines Stoffgemisches zur Herstellung eines Arzneimittels zur topischen Anwendung vorgeschlagen, welches Honig und Olivenöl enthält.

Positive Wirkungen zeigen Honig und Honigzubereitungen bei der Heilung von Wunden im Magen-Darm-Trakt, u. a. bei der Heilung von peptischem Ulcus, hervorgerufen durch Helicobacter pylori.

Honigzubereitungen wurden erfolgreich auch zur Heilung von Hämorrhoiden erprobt (US 6,482,442).

Aus der EP 0 216 557 A2 ist eine W/O-Emulsion bekannt, deren Ölphase auf Vaseline beruht. Die Emulsion dient insbesondere als Hautbefeuchtungsmittel, wobei u. a. auch Honig als Feuchthaltemittel Erwähnung findet.

In Russland sind Medizinprodukte auf Honigbasis in der Zahnmedizin u.a. zur Behandlung von Parodontose zugelassen.

Pilzerkrankungen der Hautoberfläche gehören zu den häufigsten auftretenden Hauterkrankungen. Sie werden meistens durch fakultative humanpathogene Pilze (Dermatophyten, Hefe oder Schimmelpilze) hervorgerufen. Dermatophyten lösen etwa 80 % aller Oberflächenerkrankungen aus.

Der Anteil von topischen Dermatotherapeutika an den Verordnungen von Dermatologen und Hausärzten liegt über 90 %. Neben antimikrobiell wirksamen Substanzen (u.a. auch Antibiotika) bei Behandlung von Hautpilzerkrankungen sind keratolytische Wirkstoffe, Retinoide, Benzoylperoxid oder Azelainsäure Bestandteile der Behandlung.

Die antimykotischen Eigenschaften des Honigs sind bekannt, u. a. bei der Unterstützung nach Chemo- und Strahlentherapie.

Auch im Bereich "Problemhaut" zeigten Honigzubereitungen ihre positiven Wirkungen z. B. bei der Behandlung von Psoriasis oder Tinea-Dermatosen.

Der Nachteil der oben beschriebenen Honig-Formulierungen ist, dass sie überwiegend in Form einer Salbe, einer Creme, eines Gels oder von imprägnierten Stoffen bzw. von Wundauflagen appliziert werden. Solche Formulierungen zeigten sehr niedrige Penetrationsraten durch die Haut/Schleimhaut.

Auch bei oralen Anwendungen zeigen Honig und Honigzubereitungen eine Reihe von positiven Wirkungen (u.a. systemische Wirkungen).

Bei gesunden Personen stehen oxidative und antioxidative Prozesse im Gleichgewicht. Ist dieses zu Gunsten der oxidativen Prozesse verschoben, spricht man von oxidativem Stress.

Alterungsvorgänge und viele Erkrankungen wie Morbus Alzheimer, Krebs, Rheuma, Arteriosklerose und Diabetes mellitus werden zunehmend mit oxidativem Stress in Zusammenhang gebracht.

Das kardiovaskuläre Risiko bezeichnet die Wahrscheinlichkeit, an kardiovaskulären Erkrankungen wie Herzinfarkt, Schlaganfall oder pAVK zu erkranken. Es steigt durch das Vorhandensein bestimmter kardiovaskulärer Risikofaktoren wie Hypertonie, Diabetes mellitus oder Dislipoproteinämie.

Der Honig weist antioxidative Wirkungen in erster Linie aufgrund bestimmter vorhandener Aminosäuren und Katalase auf und kann die kardiovaskulären Risikofaktoren absenken.

Honig zeigt im Vergleich zur Saccharose eine signifikante Herabsenkung von Cholesterin, LDL, Triglyceriden, C-reaktivem Protein.

An Ratten mit einer Honig-Diät wurde eine signifikant niedrigere Gewichtszunahme im Vergleich zu Tieren mit einer Saccharose-Diät nachgewiesen. Auch biochemische Stoffwechselparameter (Blutzucker, Triglyceride, Cholesterin u.a.) waren signifikant besser bei der Honig-Diät.

Die antientzündliche Wirkung von Honig und Propolis wurde in vivo und in vitro untersucht.

Eine Rezeptur aus Honig und pflanzlichen Extrakten (*Semecarpus anacardium* und *Emblica officinalis*) zeigte sich effizient bei rheumatischer Arthritis (RA) bei Ratten auf Grund einer hohen antioxidativen Wirkung und synergistisch wirkender Polyphenole, Flavonoide und Tannine. Diese Honig-Formulierung zeigt auch analgetische und antipyretische Wirkung vergleichbar mit Diclofenac.

Traditionell wird Honig weltweit als Brotaufstrich verwendet. Ein Brotaufstrich auf Honigbasis, der als W/O-Emulsion vorliegt, wobei der Honig in der Fettphase dispergiert vorliegt, wird in der GB 2 113 523 A beschrieben.

Außerdem ist er Bestandteil von Backwaren, Getränken, Pralinen und zahlreichen anderen Lebensmitteln und wird als natürliches Konservierungsmittel untersucht. Wenig bekannt ist die Anwendung von Honig in diätetischen Produkten, funktionalen Lebensmitteln und Nahrungsergänzungsmitteln.

Auch für kosmetische Anwendungen kann Honig eingesetzt werden. Basierend auf der Erkenntnis, dass die Haut bereits ab dem 20. Lebensjahr auch durch Mangel an Nährsubstraten, der durch die Degeneration der Papillarkapillaren bedingt wird, deutlich dünner wird, zu altern anfängt und somit an Vitalität verliert, besteht zur Erhaltung der "Vitalität" der Hautzellen, insbesondere der Zellen des Stratum germinativum, der Bedarf, ein wirksames Vehikel zu haben, welches die Zellen von außen mit den notwendigen zellulären Nährstoffen in ausreichender Menge versorgen kann. Zelluläres Substrat sind in erster Linie Zucker, Aminosäuren, sekundäre Nährstoffe, Mineralien und Sauerstoff. Alternde und degenerative Haut zeigt nachweislich Substrat- bzw. Sauerstoffmangel.

Erstes wichtiges zelluläres Substrat ist Glukose; auch diese kann vermutlich nicht immer von innen über die degenerierten Papillarschlingen in ausreichendem Maße angeliefert werden. Auch andere Zucker wie z. B. Glucosamin, das im Körper aus Glukose biosynthetisiert wird, nehmen mit zunehmendem Alter sehr stark ab und sollten zur Verfügung gestellt werden.

Zweites wichtiges Substrat sind Aminosäuren, um die Synthese-Leistungen der Zellen zu unterstützen.

Drittes wichtiges Substrat ist Sauerstoff zur Versorgung der Zellen mit Nährstoffen. Außerdem sollte der Sauerstoff diese Zellen über den Mechanismus der erleichterten Diffusion leicht erreichen.

Durch kompensatorische natürliche Zellversorgung und nachhaltige Feuchtigkeitssteigerung der Haut könnte die extrazelluläre Matrix durch Wiederaufbau von Glucosaminoglykanen biologisch repariert werden Die Versorgung mit essentiellen Sacchariden spielt eine wichtige Rolle bei der Biosynthese von Glucosaminoglycanen. Von besonderer Bedeutung ist auch die Steigerung der Kollagen-Synthese, die nur bei ausreichender Bioverfügbarkeit von Ascorbinsäure und Sauerstoff möglich ist. Die Steigerung der Kollagen-Synthese führt zur Straffung und Glättung der Haut.

Hierbei kommt es vor allem darauf an, die Hornschicht als Barriere zu überwinden.

Eine Verbesserung der Penetration des Wirkstoffes in der Haut steht im Vordergrund. Hier ist allerdings eine systemische Aufnahme nicht erwünscht, z. B. bei lokalwirkenden Antimykotika oder kosmetischen Zubereitungen. In diesem Fall kann der Wirkstoff in einer Konzentration eingesetzt werden, in der keine Übersättigung in wasserreicher Mikroemulsion eintritt.

Honig kann nur eine begrenzte Menge und Anzahl von wasserlöslichen Stoffen aufnehmen, ohne seine oben beschriebenen Eigenschaften zu verlieren.

Zusammen mit fettlöslichen Stoffen ist es nicht möglich, eine homogene Honig-Lösung zu erzielen. Mit Hilfe von Emulgatoren entstehen nur grobe Emulsionen, die nicht in die Haut bzw. Schleimhaut penetrieren können.

Um die effizienten Wirkungen von Honig bei medizinischen (Wundheilung, antientzündlich, cholesterinsenkend u.a.), kosmetischen bzw. dermokosmetischen Anwendungen sowie im Lebensmittelbereich zu zeigen, sollten neue HonigZubereitungen gute Haut bzw. Schleimhaut penetrierende Eigenschaften besitzen.

Sowohl für die medizinische als auch für die kosmetische Behandlung haben die Penetration und Permeation durch das Stratum corneum bis zu den vitalen Zellen der Epidermis eine große Bedeutung. Man muss in diesem Zusammenhang zwischen kosmetischer Hautpflege und kosmetischen Behandlungen unterscheiden.

Im Gegensatz zu der kosmetischen Hautpflege versteht man unter kosmetischer Behandlung, insbesondere im Rahmen des Anti-Aging-Konzeptes, die Unterstützung der vitalen Zellen mit essentiellen Nährstoffen, und somit die Wiederherstellung der funktionsschwachen Bestandteile der Haut, z. B. Förderung der Kollagen- und Hyaluronsäure-Biosyntese, antioxidative oder hormonelle Wirkungen, bei denen es sich um intrakutane Behandlungen handelt.

Die Penetration von traditionellen kosmetischen Präparaten wie Salben, Cremes, Lotionen, Gelen, Masken und Liposomen in die Haut ist sehr gering. Solche Präparate penetrieren nur in die obersten Schichten des Stratum corneum (SC) und zeigen keine Wirkungen an den lebenden Zellen des SC, der Epidermis und Dermis.

Seit den 80er Jahren ist die Anwendung von Liposomen sowohl in der Medizin als auch in der Kosmetik gut bekannt Die Liposomen besitzen eine Hülle aus einer oder mehreren Wasser-Phospholipid flüssigkristallinen Doppelschichten. Sie haben einen wässrigen Innenraum als Behälter für wasserlösliche Wirkstoffe, und sie befinden sich in einer wässrigen Lösung. Die Herstellung von Liposomen benötigt spezielle Geräte und ist sehr kostenintensiv. Liposomen können nur schlecht in das Stratum corneum eindringen

Aufgabe der vorliegenden Erfindung war, Honigzubereitungen zur Verfügung zu stellen, die eine gute Penetration der Haut/Schleimhaut zeigen und auf diese Weise Wirkstoffe effektiv an ihren Zielort bringen.

Diese Aufgabe wird erfindungsgemäß gelöst durch eine Mikroemulsion mit
- 50 bis 80 Gew.-% einer Ölphase,
- 2 bis 40 Gew.-% einer Mischung aus einem oder mehreren W/O-Emulgatoren und einem oder mehreren O/W-Emulgatoren im Verhältnis 1:5 bis 1:1,
- 5 bis 40 Gew.-% Honig, Gelee Royal, Propolis und/oder Perga,
- 0,01 bis 30 Gew.-% Co-Emulgatoren, wobei die Co-Emulgatoren ausgewählt sind aus der Gruppe der Phospholipide,
- 1 bis 20 Gew.-% Wasser oder einer wässrigen Lösung und
- einem oder mehreren wasserlöslichen oder fettlöslichen Wirkstoffen,
wobei die Wirkstoffe ausgewählt sind aus der Gruppe: Analgetika, Lokalanästhetika, Antiphlogistika, Antirheumatika, Glukokortikoide, Antibiotika, Antimykotika, Virustatika, Immunsuppressiva, Phytopharmaka, Antihistaminika, Chemotherapeutika, Durchblutung fördernde Stoffe, Steroide, Immunomodulatoren, Antipsoriatika, Keratolytika, Hormone, Alkaloide, Hormone, Ceramide.

Die wasserlöslichen oder fettlöslichen Wirkstoffe oder Arzneistoffe können in die HonigMikroemulsionen inkorporiert werden, ohne dass es zu einer Instabilität der Mikroemulsion kommt. Je nach Wasser- bzw. Fettlöslichkeit sind die Wirkstoffe Teil der kontinuierlichen oder der dispersen Phase.

Es hat sich gezeigt, dass die erfindungsgemäßen Mikroemulsionen auf Basis von Honig bzw. der weiteren Bienenprodukte Gelee Royal, Propolis und Perga sehr schnell und tief in die Haut durch das Stratum corneum eindringen und die Haut mit den o. g. Stoffen anreichern. Auf diese Weise kann der Haut Feuchtigkeit zugeführt und die Glätte der Haut verbessert werden. Darüber hinaus sind die Mikroemulsionen auch in der Lage, in die Schleimhaut einzudringen, so dass positive Wirkungen auch im Magen-Darm-Trakt, Nasen, Ohren, Mund oder an den Geschlechtsorganen entfaltet werden.

Der mit der Mikroemulsion in die Haut/Schleimhaut eingebrachte Honig bzw. die Bienenprodukte Gelee Royal, Propolis oder Perga können neben der Feuchtigkeitsregulation weitere wichtige für die Haut günstige Wirkungen entfalten. Er wirkt auf zweierlei Weisen antioxidativ, nämlich durch die enthaltenen Aminosäuren und Katalasen. Weitere Wirkungen sind die Entzündungshemmung, die antibiotische und die immunstimulierende Wirkung. Mit Hilfe der Mikroemulsion werden der Haut/Schleimhaut Arznei- und kosmetische Wirkstoffe zugeführt.

Die Mikroemulsionen können sowohl in kosmetischen und dermatologischen Produkten zur Applikation auf der Haut/Schleimhaut als auch in Produkten zur oralen Verabreichung eingesetzt werden. Hierbei kann es sich um medizinische und veterinärmedizinische Produkte, aber auch um Lebensmittel, Nahrungsergänzungsmittel oder diätetische Produkte handeln.

Ebenso möglich ist es, die Mikroemulsionen zum Versprühen vorzusehen. Denkbar sind Mikroemulsionen in flüssiger, sprühbarer, gelartiger, halbfester und fester Form.

Die Verwendung von Mikroemulsionen in Lebensmitteln, Nahrungsergänzungsmitteln und diätetischen Produkten hat verschiedene Vorteile. Die Mikroemulsion erlaubt den Schutz von empfindlichen Proteinen wie Enzymen vor der Verdauung und ihre Solubilisierung, die verbesserte Adsorption von Wirkstoffen der Schleimhaut des Magen-Darm-Traktes und antioxidative Wirkungen von öl- und wasserlöslichen Vitaminen und Antioxidantien.

Bei der erfindungsgemäßen Mikroemulsion handelt es sich um eine Wasser-in-Öl-Mikroemulsion (W/O-Mikroemulsion). Diese kann jedoch durch Umsetzung mit einer Wasserphase (Wasser oder Wasser/Lösungsmittel-Gemisch) in eine sekundäre oder eine Öl-in-Wasser-Mikroemulsion (O/W-Mikroemulsion) überführt werden. O/W-Mikroemulsionen eignen sich u. a. in besonderer Weise zum Versprühen.

Bevorzugt weist eine Mikroemulsion 0,01 bis 30 Gew.% eines oder mehrerer wasserlöslicher oder fettlöslicher Wirkstoffe, bspw. Arzneimittel auf.

Überraschenderweise wurde festgestellt, dass eine primäre W/O-Honigmikroemulsion auf Basis herkömmlicher Öle, Emulgatoren und Co-Emulgatoren hergestellt werden kann, wobei für die Herstellung von primären W/O-Honigmikroemulsionen synthetische-, halbsynthetische- und natürliche Öle verwendet werden können. Es sind sowohl nicht-ionische als auch zwitterionische Emulgatoren einsetzbar. Als Co-Emulgatoren sind insbesondere Phospholipide verwendbar.

Als Mikroemulsion werden tensidhaltige Mehrkomponentensysteme bezeichnet, die thermodynamisch stabil und transparent bis leicht opaleszierend sind. Diese Systeme sind zusammengesetzt aus einer wässrigen und einer öligen Komponente sowie einem Tensid und in der Regel einem Co-Tensid, dessen Aufgabe es ist, die Grenzflächenfluidität durch die Auflockerung des Tensidfilms zu erhöhen. Das Co-Tensid penetriert zwischen die Tensidmoleküle, wodurch sich die geometrische Packung der Tensidschicht und die geometrischen Parameter der Tropfen bzw. Packungsparameter oder ihre Krümmung verändern. Je nach Kompositionen können wasser-, öl- oder bikontinuerliche Systeme mit verschiedenen Strukturen (Mizellen, geschwollene Mizellen, strukturierte Mikrobereiche, bikontinuierliche Bereiche) entstehen.

Mikroemulsionen besitzen die einzigartige Eigenschaft, dass die Oberflächenspannung zwischen den Phasen sehr gering ist. Dadurch sind die Phasen thermodynamisch gegen Phasentrennung sehr stabil; somit sind z. B. die Mizellen in Form sehr kleiner Partikel mit einer Größe von 10 bis 200 nm (im Gegensatz zu üblichen Emulsionen mit einer Partikelgröße von 1 bis 20 µm) stabil. Gemäß der vorliegenden Erfindung liegt der Durchmesser der dispergierten Wassertröpfchen typischerweise in einem Bereich zwischen 20 und 150 nm.

Die Auswahl des Tensids entscheidet darüber, ob sich Mikroemulsionen Typ O/W (Öl-in-Wasser) oder W/O (Wasser-in-Öl) bilden. Eine Aussage darüber lässt sich u.a. aus dem HLB-Wert ableiten. Liegt dieser im Bereich zwischen 4,0 und 7,0 bilden sich W/O- Mikroemulsionen. Bei größeren Werten zwischen 9,0 und 20,0 entstehen O/W- Mikroemulsionen.

Die Tatsache, dass in Mikroemulsionen Öl und Wasser gleichberechtigt in hohen Konzentrationen in einem Einphasensystem vorliegen können, verleiht ihnen ideale Eigenschaften eines Universallösungsmittels. Sie können sowohl mit lipophilen als auch hydrophilen Stoffen verabreicht werden.

Die Charakterisierung von Mehrkomponentensystemen ist allerdings eine sehr komplexe Problematik. Meist entstehen Mikroemulsionen nur in einem eng begrenzten Gebiet innerhalb eines sog. Phasendiagramms. Bereits kleine Veränderungen in der Zusammensetzung können zu Verschiebungen der Mikroemulsionsgebiete im Phasen-Diagramm führen.

Mikroemulsionen können sehr vielseitig eingesetzt werden in verschiedenen Darreichungsformen. Die Mikroemulsionen werden je nach Wirkstoffeigenschaft formuliert und auf unterschiedlichen Applikationswegen (in der Medizin, Kosmetik oder Lebensmittelindustrie) verwendet. Im Bereich der Veterinärmedizin erlauben die Mikroemulsionen die Herstellung von Medikamenten für die perorale oder perkutane Anwendung zur Behandlung von Entzündungen, Mastitis, Gastroenteritis und die Herstellung von Mitteln zur Insektenbekämpfung.

Für die Kosmetik können Präparate mit bioregulierenden aktivierenden Stoffen (u.a. zur Durchblutungsförderung, Sauerstoffversorgung, etc.), die das Zellwachstum unterstützen (Cellulite, Anti-Aging, Hypoxie) zur Verfügung gestellt werden. Ebenso möglich ist die Herstellung von Präparaten mit bioregulierenden hemmenden Stoffen, die das Zellwachstum unterdrücken (antibakterielle Wirkung, Bekämpfung der Hautalterung, konstriktive Wirkung, anti-proliferative Wirkung, zur Behandlung von Psoriasis, Tumoren etc.). U. a. ergeben sich folgende Anwendungen:
- Anti-Aging
- Behandlung von Problemhaut (Akne, Cellulite, Psoriasis, Photodermatosen)
- Anfeuchtung
- Bleichung
- Färbung
- Haarpflege
- Behandlung von Alopecia
- Nagelpflege
- Lippenstifte
- Seife

Unabhängig vom konkreten Anwendungsbereich weisen die erfindungsgemäßen Mikroemulsionen u. a. folgende Vorzüge auf:
- hervorragende Lösungskapazität für hydrophile und hydrophobe Stoffe
- hohe (thermodynamische) Stabilität
- leichte Herstellbarkeit
- Verzicht auf Konservierungsstoffe möglich
- breites Spektrum von möglichen Anwendungen
- erleichterte Penetration durch Haut und Schleimhaut

### Ölphase

Vorteilhaft wird die Ölphase gewählt aus der Gruppe pharmazeutisch und/oder in Lebensmitteln akzeptabler Öle. Bei den Ölen kann es sich z. B. um Ester aus Alkancarbonsäuren und Alkoholen handeln. Solche Esteröle können vorteilhaft ausgewählt werden aus der Gruppe bestehend aus: Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisonanoat, 2-Ethylhexylpalmitat, 2-Ethylhexyllaurat, 2-Hexyldecylstearat, 2-Octyldodecylpalmitat, Ethyloleat, Oleyloleat, Oleylecurat, Erucyloleat sowie synthetischen, halbsynthetischen und natürlichen Gemischen solcher Ester.

Ferner kann die Ölphase ausgewählt werden aus der Gruppe der Dialkylether, der Gruppe der Alkohole, sowie der Fettsäuretriglyceride, namentlich der Triglycerinester gesättigter und/oder ungesättigter Alkancarbonsäuren einer Kettenlänge von 8 bis 24, insbesondere 12 bis 18 C-Atomen. Die Fettsäuretriglyceride können beispielsweise gewählt werden aus der Gruppe der synthetischen, halbsynthetischen und natürlichen Öle, z. B. Olivenöl, Mandelöl, Avocadoöl, Sonnenblumenöl, Sojaöl, Erdnussöl, Rapsöl, Palmöl, Kokosöl, Palmkernöl und dergleichen mehr.

Die Ölphase kann auch auf pharmazeutisch akzeptablen Ölen wie 2-Ethylhexylisostearat, Octyldodecanol, Isotridecylisonanoat, Isoeicosan, 2-Ethylhexylcocoat, Capryl-Caprinsäure-triglycerid, Dicaprylylether, Mandelöl, Avocadoöl oder Olivenöl basieren.

Des Weiteren kann die Ölphase mit schwerflüchtigen Kohlenwasserstoffen wie Paraffinöl, Squalen oder Squalan erzeugt werden. Ebenfalls möglich ist die Verwendung von Fettalkoholen mit 6 bis 18 Kohlenstoffatomen in geraden Ketten sowie von Säuren aus der Gruppe: Lauryl-, Palmitin-, Myristin-, Aradidon, Linolen- und Linolsäure.

Die erfindungsgemäße Mikro-Emulsion enthält vorteilhaft essentielle Fettsäuren, insbesondere Linol- und γ-Linolensäure, Ölsäure, Eicosanpentaensäure oder deren Derivate, Borretschöl, Nachtkerzenöl, Hagebutten-Öl, Rosa Rubignose, Centalla oder Inophyllum.

### Emulgatoren

Für die Herstellung der primären W/O-Mikroemulsion können verschiedene Typen von Emulgatoren verwendet werden, beispielsweise aus der Gruppe der ethoxylierten Fettalkohole mit 8-18 Kohlenstoffatomen in geraden Ketten, insbesondere Polyethylenglycol(2)stearylether (Steareth-2), Steareth-20, Oleth-3 oder Oleth-10.

Ferner können die Emulgatoren vorteilhaft ausgewählt werden aus der Gruppe der Sorbitanderivate wie Sorbitanmonolaurat oder Sorbitantrioleat oder aus der Gruppe der ethoxylierten Sorbitanderivate wie Polyethylenglycol-(20)sorbitanmonolaurat, Polyethylenglycol(20)sorbitanmonostearat.

Darüber hinaus können die Emulgatoren vorteilhaft ausgewählt werden aus der Gruppe der Glycerylderivate von gesättigten und ungesättigten Fettsäuren, insbesondere aus Mono-, Di-, Tri- und Polyglycerinderivaten, einschließlich Polyglyceryldiisostearat, Polyglyceryl-2-oleylether, Polyglyceryl-6-distearat, Polyglyceryl-4-oleylether.

Ebenfalls möglich ist die Verwendung von ethoxylierten Glycerylestern. Als ethoxyliertes Triglycerid kann z. B. Polyethylenglycol(20)glyceryltristearat verwendet werden. Eine weitere Möglichkeit stellen ethoxylierte Alkylether wie Polyethylenglykol-dodecylether (Brij30) oder Polyethylenglykol-hexadecylether (Brij52) dar.

Des Weiteren können die Emulgatoren ausgewählt werden aus der Gruppe der Fettalkohol-(C₁₆-C₁₈)-Glukoside. Als Alkylglukoside können vorteilhaft Sucrosestearat, Sucrosepalmitat, Plantacare 1200 UP und Plantacare 2000 UP gewählt werden.

### Co-Emulgatoren

Die Co-Emulgatoren der erfindungsgemäßen Mikroemulsionen werden ausgewählt aus der Gruppe der Phospholipide, beispielsweise:
- Lecithin aus Pflanzen (z. B. Soja, Raps, Baumwollsamen) und Eigelb,
- Phosphatidylcholin aus Soja und Eigelb,
- Phosphatidylethanolamin,
- Phosphatidylserin,
- Phospatidylinosit aus Soja, Raps, Baumwollsamen,
- hydroxyliertes Lecithin.

Vorteilhaft wird der Co-Emulgator ausgewählt aus der Gruppe: Lecithin aus Soja und Eigelb, bekannt unter den Handelsbezeichnungen Epikuron 135, Epikuron 170, Epikuron 200, Epikuron 200 SH, Phospholipon 25, NAT-8539 (Nattermann).

Die Menge der Phospholipide (einer oder mehrerer Verbindung) in der Zubereitung beträgt 0,01 bis 30 Gew.-%, vorzugsweise 0,1 bis 10 Gew.-%, insbesodere 1 bis 5 Gew.-% bezogen auf das Gesamtgewicht der Zubereitung.

Ebenfalls verwendbar als Co-Emulgatoren sind Cholesterin und Cholesterinderivate: Als ethoxyliertes Cholesterinderivat kann vorteilhaft Polyethylenglycol(10)-Sojasterol verwendet werden.

### Lösungsmittel

Für die Herstellung der primären W/O-Mikroemulsionen können z. B. die folgenden Typen von Lösungsmitteln verwendet werden:
- kurzkettige- und langkettige Alkohole (z. B. Ethanol, Propanol, Isopropanol),
- Glykole (Propylenglykol, 1,2-Octandiol, 1,2-Hexandiol),
- Glycerin, Diglycerin,
- Pyrrolidone (N-Methylpyrrolidon,N-Ethylpyrrolidon),
- Kohlenhydratderivate (Dimethyisosorbid (Arlasolve®), Sorbitol).

Vorteilhaft wird das Lösungsmittel ausgewählt aus der Gruppe: Ethanol, Isopropanol, 1,2-Octandiol, Propylenglykol, N-Methylpyrrolidon, Dimethylisosorbid.

### Wirkstoffe

Die Mikroemulsion gemäß der Erfindung enthält Wirkstoffe ausgewählt aus der Gruppe: Analgetika, Lokalanästhetika, Antiphlogistika, Antirheumatika, Glukokortikoide, Antibiotika, Antimykotika, Virustatika, Immunsuppressiva, Phytopharmaka, Antihistaminika, Chemotherapeutika, Durchblutung fördernde Stoffe, Steroide, Immunomodulatoren, Antipsoriatika, Keratolytika, Hormone, Alkaloide, Hormone, Ceramide.

Es ist gegebenfalls zusätzlich möglich und vorteilhaft, die erfindungsgemäßen Zubereitungen als Grundlage für pharmazeutische Formulierungen zu verwenden. Die Übergänge zwischen reinen Kosmetika und reinen Pharmaka sind dabei fließend.

Es ist vorteilhaft, den Zubereitungen im Sinne der vorliegende Erfindung weitere anti-irritative oder antientzündliche Wirkstoffe zuzugeben, insbesondere NSAR (nichtsteroidale Antirheumatika), Cortikosteroide, Phytopharmaka und pflanzliche Extrakte.

Medizinische topische Zusammensetzungen im Sinne der vorliegenden Erfindung enthalten in der Regel ein oder mehrere Medikamente in wirksamer Konzentration.

Im Falle der Verwendung pflanzlicher Extrakte werden diese vorteilhaft gewonnen aus: Angelikawurzel, Arnikablüten, Basilikumblätter, Birke, Borretschsamen, Chili, Curcuma longa, Curcuma xynthorrhiza, Dillsamen, Erdnuß, Fenchel, Gewürznelke, Himbeersamen, Hopfen, Ingwer, Iriswurzel, Johanniskraut, Kamillenblüten, Kardamon, Karottensamen, Kawa-Kawa, Lavendelblüten, Majoranblätter, Melissenblätter, Muskat, Myrrhe, Oreganoblätter, Paprika, Pfefferminzblätter, Ringelblüten, Rosmarin, Salbei, Sanddornsamen, Sternanis, Tanne, Thymian, Vanille Bourbon, Weihrauch, Zimtrinde. Besonders vorteilhaft sind Angelikawurzel, Arnikablüten, Birke, Borretschsamen, Curcuma longa, Curcuma xynthorrhiza, Myrrhe, Tanne, Weihrauch und Zimtrinde.

Fettlösliche Vitamine werden zweckmäßigerweise ausgewählt aus der Gruppe: Vitamin A und Derivate, Vitamin C (Ascorbinsäure) und Derivate, Vitamin E (Tocopherol) und Derivate. Beispielsweise können Tocopherolacetat und Ascorbinsäurepalmitat verwendet werden. Ebenfalls einsetzbar sind Ubichinon und seine Derivate.

Antioxidanten sind vorzugsweise ausgewählt aus der Gruppe: Aminosäuren (z. B. Glycin, Histidin, Tyrosin) und deren Derivate, Imidazole, Carotinoide, Carotine (z. B. α- und β-Carotine) und deren Derivate, Thiole (Glutathion, Thioredoxin, Cystein, Cystamin und deren Derivate), Vitamin E und dessen Derivate. Besonders vorteilhaft ist Tocopherolacetat.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew. % auf das Gesamtgewicht der Zubereitung bezogen.

Die erfindungsgemäßen Mikroemulsionen können Diffusionsverstärker wie N-Methylpyrrolidon, Dimethylisosorbid, Menthol, Pinen, Thymol, Campher, Koffein, Diethylenglykol-Ester, z. B. Diethylenglykolmonoethylester, Diethylenglykol enthalten. Vorteilhaft werden die Diffusionsverstärker ausgewählt aus der Gruppe N-Methylpyrrolidon, Dimethylisosorbid und Diethylenglykolmonoethylester.

Bei der Herstellung der primären W/O-Mikroemulsionen haben sich auch die folgenden Typen von ätherischen Ölen als vorteilhaft herausgestellt: Terpene (Mono-, Sesquiterpene, Diterpene): Citrusöl, Pinie, Kamille; Alkohole (Monoterpenole, Sesquiterpenole, Diterpenole): Ravensara, Ysop, Niaouli; Aldehyde: Melisse, Eucalyptus; Ketone (Monoterpenketone, Sesqui- und Diterpenketone): Schafgarbe, Thuja sowie Ester (Monoterpenester): Lavendelöl, Ylang Ylang; Phenole: Thymian; Phenylether: Anis, Nelke; Oxide: Cineol; Lactone: Patchouli, Weihrauch und Kumarine.

Besonders vorteilhaft werden die ätherischen Öle ausgewählt aus Citrusöl, Pinie, Kamille, Ravensara, Ysop, Niaouli, Melisse, Eucalyptus, Schafgarbe, Thuja, Lavendelöl, Ylang Ylang oder Teebaumöl.

Die Menge der ätherischen Öle (eine oder mehrere Verbindungen) in den Zubereitungen beträgt vorzugsweise 0,1 bis 10 Gew.-%, besonders bevorzugt 0,5 bis 5 Gew.-%, insbesondere 1 bis 5 Gew.-% bezogen auf das Gesamtgewicht der Zubereitung.

Essentielle Fettsäuren werden vorzugsweise ausgewählt aus Linol- und γ-Linolensäuren oder essentiellen fettsäurehaltigen Ölen.

Besonders vorteilhaft sind γ-Linolensäure, Borretschöl, Nachtkerzenöl und Fischöl.

Es hat sich herausgestellt, dass aus der primären W/O-Mikroemulsion durch Zugabe von Wasser oder wässrigen Lösungen sowohl sekundäre W/O- als auch O/W-Mikroemulsionen herzustellen sind. Diese sind besonders geeignet, wasserlösliche kosmetische und/oder pharmazeutische Stoffe aufzunehmen. In der primären oder sekundären W/O-Mikroemulsion oder der O/W-Mikroemulsion können folgende wasserlösliche Stoffe enthalten sein:
Antioxidanten wie Hydroxy- und Dihydroxybenzoate, Hippurate, Salicylate, Cystein und Derivate, Glutathion, Vitamin C und dessen Derivate (z. B. Mg-Ascorbylphosphat, Ascorbylacetat) und Vitamin H, Superoxid-Dismutase, Katalase, Polyphenole, Isoflavone. Besonders vorteilhaft sind Ascorbylacetat, Superoxid-Dismutase, Cystein und Glutathion.

Wasserlösliche Vitamine und Provitamine werden ausgewählt aus der Gruppe der Vitamin B-Komplexe, Vitamin C und Derivate, Vitamin H und Derivate, Biotin, Pantothensäure, Panthenol.

Ferner können Diffusionsverstärker wie Terpene, Cineol, Menthol, Propylenglykol, Butylenglykol, Polyethylenglykol mit 4 bis 250 Ethylenglykoleinheiten, N-Methylpyrrolidon, Dimethylisosorbid, Diethylenglykolester z. B. Diethylenglykolmonoethylester, Diethylenglykol, Oleinsäure, Säuren oder Salicylsäure verwendet werden. Besonders vorteilhaft sind N-Methylpyrrolidon, Dimetylisosorbid, Salicylsäure, Cineol, Menthol, Diethylenglykolmono-ethylester und Oleinsäure.

Saccharide und Oligosaccharide sind vorteilhafterweise ausgewählt aus der Gruppe: Glucose, Fructose, Mannose, Mannitol, Inosit, N-Acetyl-D-glucosamin, D-Glucosamin, Chito-Oligosaccharide, Trehalose. Besonders vorteilhaft sind Glucose, D-Glucosamin, N-Acetyl-D-Glucosamin, Chito-Oligosaccharide und Trehalose.

Als Polysaccharide können Chitosan, Hyaluronsäure, Heparin, Dextran, Zelluloseester oder Alginsäure in die Formulierungen eingebracht werden. Bevorzugt sind Chitosan und Hyaluronsäure.

Ferner können Proteine und Proteinderivate aus der Gruppe der Struktureiweiße wie Kollagen, Fibrin, Elastin eingesetzt werden, wobei Kollagen besonders bevorzugt ist.

Als Hormone oder hormonähnliche Substanzen sind z. B. Hydrocortison und dessen Derivate, Melatonin, Glycyrrhizinsäure und deren Derivate sowie andere pflanzliche Hormone und pflanzliche Steroide einsetzbar, bevorzugt Melatonin und Glycyrrhizinsäure.

Des Weiteren können pflanzliche Extrakte verwendet werden, insbesondere aus der Gruppe: Meristem-Extrakt, Aloe Vera, Echinacea, Hamamelis-Extrakt, Spargelextrakt, Niembaum, Polyplant-Mikroemulsion, Rosskastanie, rotes Weinlaub, Arnika, Ringelblume, Efeu, Brennnessel, Kamille, Schachtelhalm.

Ebenfalls verwendbar sind Aminosäuren, Peptide, Protein-Hydrolysate, z. B. Seidenprotein-Hydrolysat, Hefehydrolysat, Weizen-Proteinhydrolysate. Bevorzugt sind Seidenproteinhydrolysat und Hefehydrolysat.

### Zusatzstoffe

Die erfindungsgemäßen Mikroemulsionen können weitere Zusatzstoffe enthalten. Hierzu gehören Elektrolyte, insbesondere eines oder mehrere Salze mit folgenden Anionen: Chlorid, Sulfat, Carbonat, Phosphat. Auch auf organischen Anionen basierende Elektrolyte können vorteilhaft verwendet werden, beispielsweise Lactate, Acetate, Benzoate, Salicylate, Propionate, Tartrate, Citrate und andere mehr. Besonders bevorzugt sind Kaliumchlorid, Kochsalz, Magnesiumsulfat, Zinksulfat und Mischungen daraus. Ebenfalls vorteilhaft sind Salzmischungen, wie sie im natürlichen Salz vom Toten Meer auftreten. Als Kationen der Salze werden bevorzugt Ammonium-, Alkylammonium-, Alkalimetall-, Erdalkalimetall-, Magnesium-, Eisen- und Zinkionen verwendet.

Die erfindungsgemäße Mikroemulsion enthält vorteilhaft chelatierende Stoffe (Ethylendiamintetraessigsäure oder deren Salze, Deferoxamin, Histidin). Besonders vorteilhaft ist Ethylendiamintetraessigsäure (EDTA).

Die erfindungsgemäßen Mikro-Emulsionen enthalten vorteilhafterweise Feuchthaltesubstanzen, sog. NMF (natural moisturizing factor) (Glycerin, Ectoine, Sorbitol, PCA-Na, Harnstoff, Allantoin, Glukosamin, Chitosan, Chito-Oligosaccharide, Carbonsäuren, Hydroxycarbonsäuren und Dicarbonsäuren als auch Polysaccharide, Hyaluronsäure oder Aloe Vera-Extrakt). Bevorzugt werden Glycerin, Harnstoff, Sorbitol, Allantoin, PCA-Na, Milchsäure, Chito-Oligosaccharide, Hyaluronsäure, Chitosan, Aloe Vera-Extrakt.

Die erfindungsgemäßen Mikroemulsionen können auch chemische Sauerstoffträger wie organische und anorganische Peroxyde, z. B. Wasserstoffperoxid, Benzoylperoxid enthalten.

Des Weiteren sind chemische und natürliche Bleichmittel als Zusatzstoffe möglich, z. B. Hydrochinone, Kojaksäure, Arbutin, Azelainsäure, Zitronen- und Gurkensaft.

Als pharmazeutisch akzeptierter oxidierender Stoff kann z. B. Hydrochinon eingesetzt werden.

Als Konservierungsstoffe können z. B. Salicylate, Benzoate, Parabene, ätherische Öle und pflanzliche Extrakte Verwendung finden.

Im Folgenden wird die Erfindung anhand von Beispielen näher erläutert.

### Beispiel 1. Primäre W/O-Typ Honigmikroemulsion.

Die primäre W/O-Typ Honigmikroemulsion auf Basis von synthetischen Ölen wurde laut folgender Formulierung hergestellt. Angabe von Rohstoffen in % (W/W)

**Tabelle 1**

| Bestandteile | 1 | 2 | 3 | 4 | 5 | 6 |
|---|---|---|---|---|---|---|
| Honig | 15 | 23,9 | 14,7 | 19,5 | 12,0 | |
| Polyglycerin-6-oleat | | | 9,2 | 8,0 | | |
| L.A.S (Gattefosse) | | | 17,1 | 16,0 | | 18,3 |
| Span 80 | 8,0 | 11,4 | | | 10 | 9,2 |
| TWEEN 80 | 16,0 | | | | | |
| TWEEN 20 | | 22,7 | | | 22,0 | |
| Ethyloleat | | 30,0 | | 28 | | |
| Isopropylmyristat | 25,0 | | 29,0 | | | |
| Myritol | | | | | 27,0 | 25,0 |
| Phosphatidylcholin | 8,0 | 2,0 | 9,0 | | | |
| NAT (Rhône-Poulenc) | | | | 10,5 | 11,0 | 12,0 |
| Ethanol, 96% | 5,0 | | 5,0 | | 5,0 | |
| Propylenglykol | | | 5,0 | | 10 | 6,0 |
| Isopropanol | | 5,0 | | 3,0 | | 6,0 |
| Arlasolve® | | | 6,0 | 7,0 | | |
| N-Methyl-pyrrolidon | | 5,0 | | | 4,0 | |
| Wasser | 3,0 | 5,0 | 5,0 | 11,0 | 9,0 | 23,2 |

### Vorbereitung einer Tensid-Mischung.

W/O- und O/W-Emulgator (z. B. 2 ml Tween-80, 1 ml Span-80), 3 ml Isopropylmyristat und 0,6 g Phosphatidylcholin (89 %ig) wurden bei Raumtemperatur gemischt.

### Herstellung einer Basis-Honiqmikroemulsion

In die resultierende Lösung wurden 2 ml Honig und 0,5 ml destilliertes Wasser zugegeben. Die resultierende trübe Lösung wurde gerührt bis zur Entstehung einer klaren gelben mittelviskosen Lösung. Die Partikelgröße der W/O-Mikroemulsion liegt im Bereich von 30 bis 70 nm.

### Beispiel 2. Herstellung einer primären W/O-Honigmikroemulsion, die als Öl-Phase Naturöle oder deren Gemische enthält

Die W/O-Mikroemulsionen wurden ähnlich wie in Beispiel 1 Nr. 1 bis 6 (Tabelle 1) hergestellt, nur wurden anstatt IPM in Ölphase der Mikroemulsion Öl-Mischungen aus Naturölen (10 bis 30 %) individuell oder auch als Gemisch in IPM oder Ethyloleat verwendet. Es wurden folgende Naturöle eingesetzt: Mandelöl, Olivenöl, Jojobaöl, Avocadoöl, Kakaobutter, Fischöl, Borretschöl, Nachtkerzenöl, Hagebuttenöl, Linol- und Linolensäure u.a.

Die resultierende klare W/O-Mikroemulsion wies eine Partikelgröße im Bereich von 40 bis 100 nm auf.

### Beispiel 3. Herstellung einer W/O Honigmikroemulsion mit Zusatz von öligen pflanzlichen Extrakten (u. a CO₂-Extrakte)

Der Öl-Phase wurden 1 % bis 30 % ölige und/oder alkoholische Extrakte (Beinwell, Curcuma longa, Curcuma xanthophyllis, Rosskastanie, Ingwer) beigemischt. Die Honig ME wurde wie in Beispiel 1 hergestellt. Dadurch entstand eine klare Honigmikroemulsion mit einer Partikelgröße von 60-110 nm.

Weiterhin wurden auch folgende pflanzliche Extrakte in einer W/O-Honigmikroemulsion in den oben angegebenen Mengen separat oder als Mischungen eingesetzt:
Echinacea purpurea (Purpursonnenhut), Hypericum perforatum (Johanniskraut), Rosmarinus officinalis (Rosmarin), Salix sp. (Weide), Passiflora incarnat (Passionsblume), Zarpagophytum procumbens (Teufelskralle), Ginseng rad., Cisti icanin (Zistrose), Betula fol. (Birke), Vitis vinifera Fol (Weinrebe).

### Beispiel 4. Herstellung einer O/W- Mikroemulsion, die ätherische Öle enthält

Wie im Beispiel 3 beschrieben, jedoch wurden anstelle der CO₂- oder alkoholischen Extrakte ätherische Öle (bis 15 %) der Öl-Phase beigemischt. Die W/O-Mikroemulsionen wurden ähnlich wie in Beispiel 1 Nr. 1 (Tabelle 1) hergestellt, nur anstatt IPM wurden Öl-Mischungen aus ätherischen Ölen (10 %) in IPM oder Ethyloleat verwendet. Es wurden folgende ätherischen Öle eingesetzt: Citrusöl, Pinie, Lavendelöl, Ylang-Ylang, Kamille, Ravensara, Ysop, Niaouli, Anis, Nelke, Thymian, Patchuli, Weihrauch, Schafgarbe, Thuja, Melisse, Eucalyptus, Rosa rubiginosa, Innophyllum callophyllum, Linol- und Linolensäure: Borretschöl, Nachtkerzenöl, Hagebuttenöl und auch 1,4-Cinneol (20 %), Menthol (20 %) und Limonen (20 %) in Isopropylmyristat oder Ethyloleat.

Es entstanden klare bis leicht opaleszierende Honigmikroemulsionen.

### Beispiel 5. Herstellung einer W/O- Mikroemulsion, die fettlösliche und wasserlösliche Vitamine und Provitamine enthält

Folgende fettlösliche Vitamine (Vitamine E, A, D) wurden separat oder als Mischungen in einer Öl-Phase, in Konzentrationen von 0,01 bis 10 % aufgelöst. Die primäre W/O-Honigmikroemulsion wurde durch die Gabe von Honig und Wasser oder wässrige vitaminhaltige Lösungen, so wie im Beispiel 1 (Nr. 1 bis 5) beschrieben gemischt. Die Partikelgrößen der W/O-Mikroemulsion liegen im Bereich von 50 bis 100 nm.

### Beispiel 6. Die W/O-Honigmikroemulsion, die Polysaccharide und Oligosaccharide enthält

Die primären W/O-Honigmikroemulsionen aus Beispiel 1 (Nr. 1 bis 5) wurden mit 0,1 bis 10 %igen wässrigen Chito-Oligosaccharid-Lösungen (Oligopharm Inc., Rußland, Korea) in sekundäre W/O- oder O/W-Honigmikroemulsionen umgewandelt. Man erhielt eine mittelviskose Mikroemulsion

Die primären W/O-Honigmikroemulsionen wurden auch durch die Gabe von wässrigen oligosaccharid- und/oder polysaccharidhaltigen Lösungen aus der Reihe Oligosaccharide (Trehalose, Fructo-, Manno-, Chitooligosaccharide), Polysaccharide u.a. Hyaluronsäure, Heparin, Chitosan, Dextran, Xanthan, Hydroxypropylzellulose und andere Zellulosederivate, Carragenan, Pektin, Aloe Vera hergestellt.

### Beispiel 7. Herstellung einer W/O- und O/W-Honigmikroemulsion, die wässrige oder alkoholische Pflanzenextrakte und (oder) mikrobiologische Extrakte enthält

Die W/O-Mikroemulsionen wurden ähnlich wie in Beispiel 1 Nr. 1 bis 6 (Tabelle 1) hergestellt. Anstelle von Wasser wurden folgende pflanzliche Extrakte oder deren Mischungen eingesetzt: Sonnenhut (Echinacea), Grüner Tee, Hamamelis, Niembaum, Aloe vera, Birke, Rosskastanie, Johanniskraut, Weinrebe, Weide, Beinwell, Hundsrose, Gingko, Weintraube, Linde, Minze, Kamille, Blaubeere, Himbeere, etc.

Die O/W-Honigmikroemulsionen werden durch Zugabe von einem Teil einer primären W/O-Honigmikroemulsion (Nr. 1 bis 6 aus Beispiel 1) mit 2 Teilen pflanzlicher Extrakte gemischt. Dadurch entstand eine klare leicht opaleszierende O/W-Honigmikroemulsion mit Partikelgrößen von 70-120 nm.

### Beispiel 8. W/O- und O/W-Honigmikroemulsion, die Hormone oder hormonähnliche Substanzen enthält: Glucocorticoide. Mineralcorticoide, Androgene. Oestrogene, Insulin. Calcitonin. Thyroxin, Prolactin, Somatotropin. Oxitocyn, Progesteron, Adrenalin, Erythropoetin, Phvtosterole.

Eine 10%ige Hydrocortisonlösung in N-Methylpyrrolidon wurde in einer Öl-Phase, in Konzentrationen von 0,01 bis 3,0 % aufgelöst. Die primären W/O-Honigmikroemulsionen wurden durch die Gabe von Honig und Wasser oder wässrige vitaminhaltige Lösungen, so wie es im Beispiel 1 (Nr. 1 bis 5) beschrieben wurde, gemischt.

Dadurch entstand eine klare, hydrocortisonhaltige Honigmikroemulsion mit Partikelgrößen von 35-65 nm.

### Beispiel 9. W/O- Honigmikroemulsion, die Lokalanästhetika enthält

Zu der Öl-Phase (Nr. 2, Tabelle 1) wurden 2,5 g Lidocaine-Base zugeben und bis zur vollständigen Auflösung von Lidocaine gerührt. Weitere Herstellungsschritte gleichen dem Beispiel 2, Tabelle 1. Anstelle von Wasser wurde jedoch eine 5 %ige wässrige Lidocain x HCl-Lösung eingesetzt. Dadurch entstand eine klare mittelviskose Honigmikroemulsion mit Partikelgrößen von 60-110 nm. Auf ähnliche Weise lassen sich Mikroemulsionen mit Prilocain, Procain, Benzocain. Morphin, Codein, Dihydrocodein, Methadon, Clofenadon oder Pentazocin herstellen.

### Beispiel 10. O/W-Diclofenac-Honigmikroemulsion

Anstatt Propylenglykol (Beispiel 5, Tabelle 1) wurde 10 %-iges Diclofenac Na in Propylenglykol zu einer Öl/Tensid-Mischung zugegeben. Die weiteren Verarbeitungsschritte werden wie im Beispiel 5 (Tabelle 1) durchgeführt. Dadurch entstand eine klare Honig-Diclofenac-Mikroemulsion mit Partikelgrößen von 90-150 nm. Auf analoge Weise lassen sich Mikroemulsionen mit Indometacin, Ibuprofen, Ketoprophen, Piroxicam, Acetylsalicylsäure oder Metatrexat herstellen.

### Beispiel 11. W/O-Honigmikroemulsion mit Wirkstoffen für eine photodynamische Therapie

Die W/O-Honigmikroemulsionen wurden ähnlich wie in Beispiel 1 Nr. 1 bis 6 (Tabelle 1) hergestellt, wobei anstatt Wasser 0,01-1,0 % wässrige Lösungen der folgenden Photosensibilisatoren eingesetzt wurden: 5-Aminolävulinsäure, Methyl-5-amino-4-oxopentanoat (MAOP) und andere Derivate von 5-Aminolävulinsäure, Porphyrine, u. a. Hämatophorphyrin (Photofrin®), Photoporphyrin IX (PP9), Chlorin, Phtalocyanide (z. B. Sulphophtalocyanin - Phtalosens-Lio®).

### Beispiel 12. W/O-Honigmikroemulsion mit antibakteriellen bzw. antifungiziden Wirkstoffen

Anstatt Propylenglykol (Beispiel 5, Tabelle 1) wurde 10 %iges Ciclopiroxamin in Ethanol zu einer Öl/Tensid-Mischung zugegeben Die weiteren Verarbeitungsschritte werden wie im Beispiel 5 (Tabelle 1) durchgeführt. Dadurch entstand eine klare Ciclopirox-Honigmikroemulsion mit Partikelgrößen von 90-130 nm. Auf ähnliche Weise lassen sich Mikroemulsionen mit Terbinafin, Clotrimazol, Nystatin u. a. herstellen.

### Beispiel 13. Physikalische Eigenschaften einer sekundären W/O- und einer O/W-Honigmikroemulsion

Honigmikroemulsion Nr. 3 (Tabelle 1) wurde mit verschiedenen Mengen von Wasser zusammengerührt. Es bilden sich klare transparente oder leicht opaleszierende sekundäre W/O- oder O/W-Honigmikroemulsion (Tabelle 2).

**Tabelle 2: Physikalische Eigenschaften der W/O- und O/W-Honigmikroemulsionen.**

| | 7 | 8 | 9 | 10 | 11 |
|---|---|---|---|---|---|
| Honigmikroemulsion, ml | 3,0 | 3,0 | 3,0 | 3,0 | 3,0 |
| Wasser, ml | 1,0 | 3,0 | 4,0 | 6,0 | 12,0 |
| Optische Eigenschaften | Klare ME | Klare ME | Klare ME | Klare ME | Klare ME |
| dynamische Viskosität ¹⁾, mPa.s | 120±13 | 70±9,5 | 40±2,4 | 24±2,1 | 18±1,5 |
| Osmolarität ²⁾, (mosmol/kg) | 320,0±0,26 | 310,0±0,18 | 290±0,19 | 280±0,25 | 280±0,3 |
| Partikelgröße ³⁾, nm, | 50±5 | 76±6,7 | 98±8,7 | 103±11,0 | 112±10,6 |

| | | | | | |
|---|---|---|---|---|---|
| 1) Rotationsviskosimeter Fluids Spektrometer RFS II (Rheometric Scientific), Scherrate 0,1 bis 100 (1/s) 2) Colloid Osmometer (Fa. Knauer) 3) Dynamische Lichtstreuung (Zetasizer NanoZS90, Fa. Malvern) | | | | | |

### Beispiel 14. Nachweis der Eindringtiefe von Honigmikroemulsionen in die Haut

Schweineohren wurden ohne Hautverletzungen sofort nach der Schlachtung aus einem Schlachthof abgeholt. Die Ohren wurden gründlich mit milder Seife abgewaschen. Unmittelbar danach wurde die subkutane Fettschicht mit dem Skalpell abgetrennt. Die Haut wurde in einem Container bei -20°C für weitere Untersuchungen aufbewahrt. Die Trennung der Epidermis von der Dermis erfolgte vorsichtig nach Erwärmung bei 60°C (1 Minute) .

Die Durchführung der Penetrationsuntersuchungen erfolgte mit Hilfe einer Diffusionzelle nach Franz mit einer effizienten Diffusionsfläche von 0,75 cm² (Crown Glass Co, Inc.).

1 ml Mikroemulsion wurde in den Donorteil der Zelle eingegeben. Als Akzeptorflüssigkeit wurde isotonische NaCI-Lösung verwendet. Nach einer definierten Zeit wurde ein Aliquot (0,5 ml) genommen und mit gleicher Menge von PBS-Puffer ergänzt.

Die Eindringtiefe von Mikroemulsionen wurde im Rahmen eines Schälverfahrens an einer Modellhaut bestimmt. Die Mikroemulsionen (Beispiel 10) wurden auf die Hautoberfläche der Ohren aufgetragen und etwa 1 bis 2 Minuten in die Haut einmassiert. Währenddessen wurden die Ohren bei 35 °C temperiert.

Für die Schälung wurden 15 Tesafilmstreifen (Typ Tesa® Office, Beiersdorf, DE) auf eine Größe von 1,5 cm ^{∗} 1,5 cm geschnitten und ausgewogen. Die auf dem Schweine-Ohr ausgewählten Untersuchungsfelder von 2 cm ^{∗} 2 cm wurden nacheinander mit den vorbereiteten Tesafilm-Streifen beklebt und danach abgezogen.

Die Streifen wurden mit Acetonitril bei 60°C im Laufe von 60 Minuten extrahiert. Die Lösungen wurden durch 0,5 µm Membrane filtriert und mit Hilfe von HPLC analysiert. Das Shimadzu LC-20A HPLC-System bestand aus einer Prevail™ Carbohydrate ESHPLC-Säule und Nucleosil 100-5 C₁₈-Säule, Verdampfungs-Lichtstreudetektor Sedex 75 (Sedere, Frankreich).

**Tabelle 3 Mittelwerte der Wiederfindung aus 4 behandelten Schweinehäuten der Honigmikroemulsion (Beispiel 10) in einzelnen Hautschichten nach 4 Stunden Applikation.**

| Verteilung von | Gesamter Zucker, µg/cm² | Diclofenac, µg/cm² |
|---|---|---|
| Hornschicht | 5,2 | 12,3 |
| Epidermis | 30,4 | 6,1 |
| Dermis | 21,3 | 5,0 |

Wie ersichtlich ist, erfolgt mit den erfindungsgemäßen Honigmikroemulsionen ein erheblich größeres Eindringen in tiefere Hautschichten, so dass Wirkstoffe besser und effektiver wirken können.

### Beispiel 15. Nachweis der Wirksamkeit der Kosmetikbehandlung mit W/O-Honigmikroemulsion.

Der Epikutantest mit W/O-Honigmikroemulsion (Beispiel 3) an 40 Probandinnen aller Hauttypen, darunter 13 Hautallergiker, hat ergeben, dass in keinem Fall eine unerwünschte Reaktion aufgetreten ist.

Die kosmetische Behandlung mit W/O Honigmikroemulsion (Beispiel 3) fand über 6 Wochen an insgesamt 15 freiwilligen Probandinnen im Alter zwischen 45 und 65 Jahren mit trockener, gealterter Haut statt. Es wurden 0,5 ml Honigmikroemulsion auf Stirn und Mundwinkelbereich mit Hilfe eines Sauerstoffsprühers (Fa. MedDrop Technology AG, St. Gallen, Schweiz) aufgetragen, leicht in die Haut einmassiert und mit 90 %-igem Sauerstoff innerhalb 15 Minuten begast. Die Behandlung fand zwei mal die Woche statt. Alle Probandinnen wurden mittels SELS-Verfahren im Untersuchungsraum vermessen. Die Messungen fanden an der Stirn und im Mundwinkelbereich statt. Es wurden die Hautparameter sowie Schuppigkeit, Rauhigkeit, Faltigkeit und Glätte mit Hilfe eines optischen 3D-Messgerätes (Fa. Courage & Khazaka, Köln), sog. SELS-Verfahren gemessen. An 14 Probandinnen wurde eine hautglättende Wirkung durch Honigmikroemulsion festgestellt (Tabelle 4).

**Tabelle 4. Hautglättungsparameter der Haut vor und nach Behandlung mit Honigmikroemulsion (in SELS-SE- Einheiten)**

| | Hautbereich | |
|---|---|---|
| | Stirn | Mundwinkel |
| | **Rauhigkeit** | |
| vorher | 0,6 ± 0,08 | 0,7 ± 0,09 |
| nachher | 0,5 ± 0,10 | 0,4 ± 0,14 |

| | **Schuppigkeit** | |
|---|---|---|
| vorher | 0,9 ± 0,17 | 0,85 ± 0,15 |
| nachher | 0,55 ± 0,12 | 0,6 ± 0,13 |

| | **Glätte** | |
|---|---|---|
| vorher | 6,5 ± 1,0 | 4,4 ± 0,9 |
| nachher | 8,3 ± 1,1 | 7,0 ± 1,1 |

| | **Faltigkeit** | |
|---|---|---|
| vorher | 8,1 ± 1,0 | 9,2 ± 1,2 |
| nachher | 7,1 ± 0,9 | 7,3 ± 0,8 |

### Beispiel 16. O/W Honigmikroemulsiojn zur Hautbehandlung von Cellulitis.

Ein Teil einer primären W/O- Mikroemulsion aus Beispiel 3 wurde mit 3 Teilen einer wässrigen 0,1 %igen Lösung von Theobromin, Nikotinsäure und Coffein gemischt. Dadurch entstand eine klare Honigmikroemulsion mit Partikelgrößen von 60-130 nm.

Es wurden 18 Probandinnen in einer drei- und fünfwöchigen Kur behandelt. Die Behandlung fand drei mal pro Woche an der Außenseite eines Oberschenkels statt. Die Gegenseite blieb unbehandelt und diente als Kontrolle. Die Hautelastizität wurde mittels Cutometer "Derma-Lab-Systems" gemessen.

**Tabelle 5 Relative Zunahme der Hautelastizität (ΔE/Eo) an 18 Probandinnen nach 3 und 5 Wochen Behandlung im Vergleich zu unbehandelten Hautstellen.**

| Behandlungsdauer | Behandlung | Hautelastizität, Δ E/Eo, % |
|---|---|---|
| 3 Wochen | ohne | 2,1 ± 0,3 |
| | mit | 32,3 ± 0,5 |
| 5 Wochen | ohne | 2,7 ± 0,2 |
| | mit | 41,1 ± 0,6 |

Die Hautelastizität nach 5 Wochen Behandlung ist um 40% gestiegen.

### Beispiel 17. W/O- und O/W-Honigmikroemulsion mit pflanzlichen antientzündlichen Substanzen

Die Behandlung von Patienten mit Erkrankungen mit entzündlichem Hintergrund wurde in den Wintermonaten 2007 in einer Rheumapraxis durchgeführt. Alle Behandlungen wurden unter Anwendung von W/O-Honigmikroemulsion (Beispiel 3) durchgeführt. Die Mikroemulsion wurde in einer Menge von 0,3 bis 0,5 ml auf die betroffene Stelle mit dem Sprüher (Sauerstoffgenerator, Fa. MedDrop Technology AG, St. Gallen, Schweiz) aufgetragen und leicht einmassiert. Danach wurde die betroffene Stelle zusätzlich mit dem Sauerstoffgas- (Sauerstoffgenerator, Fa. MedDrop Technology AG, St. Gallen, Schweiz) im Laufe von 20 Minuten begast. Die Behandlungen fanden normalerweise 2 bis 3 mal pro Woche statt. Die Erfolge wurden mit Hilfe der visuellen Analog-Skala (VAS) ermittelt und als Verringerung der Schmerzen erfasst.

**Tabelle 6 Indikationen, Fallzahl und Behandlungserfolge**

| Indikation | Patientenanzahl | erfolgreich | erfolglos |
|---|---|---|---|
| Arthrose im oberen Sprunggelenk | 8 | 7 | 1 |
| Arthritis im Kniegelenk | 6 | 4 | 2 |
| Arthritis in der Hüfte | 7 | 6 | 1 |
| Handgelenkschmerzen | 4 | 3 | 1 |
| Halswirbelsäulensyndrom | 5 | 4 | 1 |
| Wirbelsäulensyndrom | 9 | 6 | 3 |
| Lendenwirbelsäulensyndrom | 12 | 7 | 5 |
| Insgesamt | 51 | 37 | 14 |

Die Tabelle zeigt, dass ein Erfolgsanteil von 67 % erreicht wurde.

### Beispiel 18. Schmerzlindernde Honigmikroemulsion

Die Wirkung der schmerzlindernden Honigmikroemulsion (aus Beispiel 9) wurde an 12 freiwilligen Probanden (4 männliche und 8 weibliche Personen im Alter zwischen 22 und 35 Jahren) getestet. Die Honigmikroemulsion wurde im Vergleich zu der EMLA®-Creme (AstraZeneca) untersucht. Es wurden etwa 2 g EMLA®-Creme und 0,4 ml Honigmikroemulsion auf 10 cm² Haut aufgetragen. Nach unterschiedlichen Einwirkungszeiten wurde das Schmerzempfinden durch kleine Nadelstiche überprüft. Die beteiligten Personen haben ihre Schmerzempfindung auf einer Schmerzgradskala eingeschätzt (0 - kein Schmerz , 5 - normaler Schmerz und 10 - schwerwiegender Schmerz).

**Tabelle 7 Zeit - Analgetisches Potential der Honigmikroemulsion und EMLA®-Creme.**

| Vehikel | Zeit, Min | Schmerzgrad |
|---|---|---|
| Honigmikroemulsion | 15 | 8,0 |
| | 30 | 6,5 |
| | 45 | 2,0 |
| | 60 | 2,0 |
| EMLA®-Creme | 15 | 9,5 |
| | 30 | 9,0 |
| | 45 | 6,0 |
| | 60 | 3,0 |

Die Honigmikroemulsion zeigt eine moderate Analgesie nach 30 Minuten und effektive Analgesie nach 45 Minuten, während EMLA®-Creme - erst nach 1 Stunde wirkt.

### Beispiel 19. Therapie von erkrankten Pferden mit einem entzündlichen Hintergrund

Alle Behandlungen wurden unter Anwendung von W/O-Honigmikroemulsion (Beispiel 3) durchgeführt. Die Mikroemulsion wurde in einer Menge von 0,5 bis 1,0 ml auf die betroffene Stelle mit dem Sprüher (Sauerstoffgenerator, Fa. MedDrop Technology AG, St. Gallen, Schweiz) aufgetragen. Danach wurde die betroffene Stelle zusätzlich mit Sauerstoffgas (Sauerstoffgenerator, Fa. MedDrop Technology AG, St. Gallen, Schweiz) im Laufe von 20 Minuten begast. Die Behandlungen fanden normalerweise 2 bis 3 mal pro Woche statt.

**Tabelle 8.Therapie von 84 erkrankten Pferden mit einem entzündlichem Hintergrund**

| Indikation | Krankheitsfälle | erfolgreich | erfolglos |
|---|---|---|---|
| Hufgelenksentzündungen | 12 | 10 | 2 |
| Fesselgelenksentzündungen | 19 | 18 | 1 |
| Gleichbeinerkrankung | 27 | 25 | 2 |
| Vorderfußwurzelentzündung | 3 | 3 | 0 |
| Fesselträgerschäden | 5 | 5 | 0 |
| Chronische Phlegmone | 2 | 2 | 0 |
| Spat | 8 | 6 | 2 |
| Sprunggelenksentzündung | 8 | 8 | 0 |
| Gesamt (in %) | 84 | 77 **(92)** | 7 **(8)** |

Die Tabelle zeigt, dass ein Erfolgsanteil von 92 % erreicht wurde.

### Beispiel 20. Milchgetränk auf Basis einer Chitooligosaccharid-Honigmikroemulsion

Es wurde die antibakterielle Wirkung der Mikroemulsion in einer Konzentration von 0,5 bis 5 % in Milch auf gram-positive Bakterien *(Listeria monocytogenes)* und gram-negative Bakterien *(Escherichia coli 157:H7)* (Beispiel 6) untersucht.

Honigmikroemulsion (2 %) in Mich (mit 3,5 % Fett) inhibiert das komplette Wachstum von *Listeria* und *E. coli* innerhalb von sieben Tagen.

50 Probanden fanden den Geschmack des Milchproduktes gut, 6 Probanden haben das Milchprodukt negativ bewertet.

### Beispiel 21. Nahrungsergänzungsmittel auf Basis von Chitooligosaccharid-/Natursäften-Honigmikroemulsionen

Honigmikroemulsion aus Beispiel 11 wurde in eine Gelatinekapsel eingeschlossen (250 µl Mikroemulsion pro Kapsel).

Das Produkt **(Beispiel 6)** enthält Chitooligosaccharide (COS), natürlichen Kürbis-, Karotten- und Jerusalem-Artischockensaft . Neben Honig liefert dieser Komplex wertvolle Substanzen für die Prophylaxe von Leberfunktionsstörungen. Chitooligosaccharide binden und entfernen toxische Substanzen. Darüber hinaus sind COS selbst effiziente Stimulatoren und Regulatoren der Leberzellfunktionen.

Die Wirksamkeit der Honigmikroemulsion (Beispiel 6) bei Patienten mit Störungen der Leberfunktion und die Beschleunigung der Sanierung geschädigter Hepatozyten wurde durch eine klinische Studie in einem Zeitraum vom 24.01.2007 bis 01.03.2007 bewiesen.

Die Diagnose einer alkoholischen Hepatitis wurde bei 22 Patienten im Alter von 24 bis 40 Jahren nachgewiesen anhand der Anamnese-Daten über häufige Alkoholexzesse, Auftreten eines Zytolyse-Phänomens, hohe Werte von Gammaglutamattranspeptidase (GGTP), Alanin-Aminotransferase (ALA), Leber- und Milzvergrößerung und Gelbsucht. Die Verumgruppe bestand aus 12 männlichen Patienten, die Kontrollgruppe bestand aus 10 Patienten mit ähnlichen klinischen und labortechnischen Daten einer Lebererkrankung. Den Patienten wurde verordnet, 2 Tabletten 2 mal täglich einen Monat lang einzunehmen.

Bei 6 Patienten wurde die Diagnose einer toxisch-allergischen (medikamentösen) Hepatitis festgestellt. Ihre Anamnese zeigte, dass sie kurz vor der Krankheit Präparate eingenommen hatten, die Sulfonamide, fiebersenkendes Febrifugal und antimykotische Medikamente enthielten. In diesen Fällen wurden Fieber, dyspeptische Anzeichen, Hautjucken in unterschiedlicher Intensität und pustulöser Hautausschlag als klinische Symptome registriert.

Die Studie ergab folgende Ergebnisse:
- das Ausschalten der dyspeptischen Anzeichen begann 5-6 Tage früher bei Patienten, die das Honigmikroemulsionspräparat nahmen;
- die Normalisierung der Bilirubinanämie begann in der Verumgruppe 6-8 Tage früher;
- das Hautjucken verschwand bei allen Patienten in der Versuchsgruppe am 14.-16. Tag der Therapie, in der Kontrollgruppe am 15.-20. Tag. Lediglich 2 Patienten hatten es mehr als 30 Tage.

**Tabelle 9. Durchschnittliche biochemische Blutparameter der Patienten vor und nach Behandlung mit der Honigmikroemulsion**

| **Messparameter** | **Normalwert** | **Verumgruppe** | | **Kontrollgruppe** | |
|---|---|---|---|---|---|
| | | **vor** | **nach** | **vor** | **nach** |
| Bilirubin | 8,0 | 164 ± 12 | 35 ± 9 | 158 ± 11 | 121 ± 13 |
| **ALA** | 0,75 | 3,8 ± 0,8 | 1,2 ± 0,6 | 4,0 ± 0,6 | 2,7 ± 0,8 |
| **GGTP** | 400 | 2680 ± 18 | 700 ± 20 | 2800 ± 20 | 1350 ± 25 |

| | | | | | |
|---|---|---|---|---|---|
| **ALA-** Alanin-Aminotransferase, **GGTP** - Gammaglutamattranspeptidase | | | | | |

Die positive Wirkung des Produktes liegt in der Fähigkeit sowohl exogene als auch endogene Intoxikationen zu stoppen und somit einer Schädigung der Leber durch Alkohol und Medikamente vorzubeugen. Das an COS, Flavonoiden und Carotinoiden reiche Produkt ist in der Lage, die Hepatozytenmembran zu schützen.

## Patentansprüche

1. Haut und/oder Schleimhaut penetrierende Mikroemulsion, enthaltend:
- 50 bis 80 Gew.-% einer Ölphase,
- 2 bis 40 Gew.-% einer Mischung aus einem oder mehreren W/O-Emulgatoren und einem oder mehreren O/W-Emulgatoren im Verhältnis 1:5 bis 1:1,
- 5 bis 40 Gew.-% Honig, Gelee Royal, Propolis und/oder Perga,
- 0,01 bis 30 Gew.-% Co-Emulgatoren wobei die Co-Emulgatoren ausgewählt sind aus der Gruppe der Phospholipide,
- 1 bis 20 Gew.-% Wasser oder eine wässrige Lösung und
- 0,01 bis 30 Gew.-% eines oder mehrerer wasserlöslicher oder fettlöslicher Wirkstoffe,
wobei die Wirkstoffe ausgewählt sind aus der Gruppe: Analgetika, Lokalanästhetika, Antiphlogistika, Antirheumatika, Glukokortikoide, Antibiotika, Antimykotika, Virustatika, Immunsuppressiva, Phytopharmaka, Antihistaminika, Chemotherapeutika, Durchblutung fördernde Stoffe, Steroide, Immunomodulatoren, Antipsoriatika, Keratolytika, Hormone, Alkaloide, Hormone, Ceramide.

2. Mikroemulsion nach Anspruch 1, **dadurch gekennzeichnet, dass** die Ölphase durch Alkancarbonsäureester, Dialkylether, Alkohole, Fettsäuretriglyceride und/oder schwerflüchtige Kohlenwasserstoffe gebildet wird.

3. Mikroemulsion nach Anspruch 1 oder 2, **dadurch gekennzeichnet, dass** die Mikroemulsion 0,01 bis 25 Gew.-% wasserlösliche und/oder fettlösliche Lösungsmittel enthält.

4. Mikroemulsion nach Anspruch 3, **dadurch gekennzeichnet, dass** die Lösungsmittel ausgewählt sind aus der Gruppe: ein- oder mehrwertige Alkohole, Polyole, Pyrrolidone, Kohlenhydratderivate.

5. Mikroemulsion nach einem der Ansprüche 1 bis 4, **dadurch gekennzeichnet, dass** die W/O- und O/W-Emulgatoren nicht-ionisch sind und die W/O-Emulgatoren einen HLB-Wert von 2 bis 7 und die O/W-Emulgatoren einen HLB-Wert von 9 bis 18 aufweisen.

6. Mikroemulsion nach Anspruch 5, **dadurch gekennzeichnet, dass** die W/O- und O/W-Emulgatoren ausgewählt sind aus der Gruppe: ethoxylierte Fettalkohole, Sorbitanderivate, ethoxylierte Sorbitanderivate, Glycerylderivate von gesättigten oder ungesättigten Fettsäuren wie Mono-, Di-, Tri- und Polyglycerylderivate, ethoxylierte Glycerylester, ethoxylierte Alkylether, Fettalkohol-(C₁₆-C₁₈)-Glucoside.

7. Mikroemulsion nach einem der Ansprüche 1 bis 6, **dadurch gekennzeichnet, dass** die Phospholipide ausgewählt sind aus der Gruppe: Lecithine, Phosphatidylethanolamin, Phosphatidylserin, Phosphatidylinosit, hydroxyliertes Lecithin.

8. Mikroemulsion nach einem der Ansprüche 1 bis 7, **dadurch gekennzeichnet, dass** die Mikroemulsion 1 bis 20 Gew.-% eines oder mehrerer wasserlöslicher oder fettlöslicher Wirkstoffe enthält.

9. Sekundäre W/O-Mikroemulsion oder O/W-Mikroemulsion, bestehend aus einer Mischung einer Mikroemulsion nach einem der Ansprüche 1 bis 8 und Wasser oder einer wäßrigen Lösung im Verhältnis 1:1 bis 1:100.

10. Mikroemulsion nach einem der Ansprüche 1 bis 9 zur Verwendung als Medikament.

11. Nicht-therapeutische Verwendung einer Mikroemulsion nach einem der Ansprüche 1 bis 10 zur Hautpflege, zur Feuchtigkeitsregulation der Haut und/oder zur Behandlung von Hautalterungserscheinungen.

## Claims

1. Skin and/or mucous membrane penetrating microemulsion comprising:
- 50 to 80% w/w of an oil phase,
- 2 to 40 % w/w of a mixture of one or several W/O emulsifiers and one or several O/W emulsifiers at a ratio of 1:5 to 1:1,
- 5 to 40 % w/w of honey, royal jelly, propolis and/or perga
- 0.01 to 30 % w/w of co-emulsifiers, wherein the co-emulsifiers originate from the group of phospholipids
- 1 to 20 % w/w of water or an aqueous solution and
- 0.01 to 30 % w/w of one or several water-soluble or fat-soluble active ingredients,
wherein the active ingredients are selected from the group: analgetics, local anesthetics, antiphlogistics, antirheumatics, glucocorticoids, antibiotics, antimycotics, virustatics, immunosuppressive agents, phytopharmaceuticals, antihistamines, chemotherapeutics, blood circulation stimulants, steroids, immunomodulators, antipsoriatics, keratolytics, hormones, alkaloids, hormones, ceramides.

2. Microemulsion according to claim 1, **characterized in that** the oil phase is formed by alkane carboxylic acid ester, dialkyl ether, alcohols, fatty acid triglycerides and/or low-volatile hydrocarbons.

3. Microemulsion according to claim 1 or 2, **characterized in that** the microemulsion comprises 0.01 to 25 % w/w of water-soluble and/or fat-soluble solvents.

4. Microemulsion according claim 3, **characterized in that** the solvent is selected from the group of mono- and polyvalent alcohols, polyols, pyrrolidones, carbohydrate derivatives.

5. Microemulsion according to any of claims 1 to 4, **characterized in that** the W/O and O/W emulsifiers are non-ionic and the W/O emulsifiers have an HLB value of between 2 and 7 and the O/W emulsifiers have an HLB value ranging between 9 and 18.

6. Microemulsion according to claim 5, **characterized in that** the W/O and O/W emulsifiers are selected from the group: ethoxylated fatty alcohols, sorbitan derivatives, ethoxylated sorbitan derivatives, glyceryl derivatives of saturated or unsaturated fatty acids such as mono-, di-, tri- and polyglycerol derivatives, ethoxylated glyceryl ester, ethoxylated alkyl ether, fatty alcohol (C₁₆-C₁₈) glucosides.

7. Microemulsion according any of claims 1 to 6, **characterized in that** the phospholipids are selected from the group: Lecithins, phosphatidyl ethanol amine, phosphatidyl serin, phosphatidylinositol, hydroxylated lecithin.

8. Microemulsion according to any of claims 1 to 7, **characterized in that** the microemulsion comprises 1 to 20 % w/w of one or several water-soluble or fat-soluble active ingredients.

9. Secondary W/O microemulsion or O/W microemulsion consisting of a mixture of a microemulsion according to any of claims 1 to 8 and water or an aqueous solution at a ratio of 1:1 to 1:100.

10. Microemulsion according to any of claims 1 to 9, for use as a medicament.

11. Non-therapeutic use of a microemulsion according to any of claims 1 to 10 for skin care, moisture regulation of the skin and/or treatment of skin aging symptoms.

## Revendications

1. Microémulsion pénétrant la peau et/ou une muqueuse contenant :
- 50 à 80 % en poids d'une phase huileuse,
- 2 à 40 % en poids d'un mélange d'un ou plusieurs émulsifiants eau/huile et d'un ou plusieurs émulsifiants huile/eau en un rapport de 1:5 à 1:1,
- 5 à 40 % en poids de miel, de gelée royale, de propolis et/ou de perga,
- 0,01 à 30 % en poids de co-émulsifiants, les co-émulsifiants étant choisis dans le groupe des phospholipides,
- 1 à 20 % en poids d'eau ou d'une solution aqueuse et
- 0,01 à 30 % en poids d'un ou plusieurs
principes actifs hydrosolubles ou liposolubles, les principes actifs étant choisis dans le groupe : analgésiques, anesthésiques locaux, antiphlogistiques, antirhumatismaux, glucocorticoïdes, antibiotiques, antimycosiques, antiviraux, immunosuppresseurs, produits phytopharmaceutiques, antihistaminiques, produits chimiothérapeutiques, produits favorisant la circulation sanguine, stéroïdes, immunomodulateurs, antipsoriasiques, kératolytiques, hormones, alcaloïdes, hormones, céramides.

2. Microémulsion selon la revendication 1, **caractérisée en ce que** la phase huileuse est formée par des esters d'acides alcanecarboxyliques, des dialkyléthers, des alcools, des triglycérides d'acides gras et/ou des hydrocarbures peu volatils.

3. Microémulsion selon la revendication 1 ou 2, **caractérisée en ce que** la microémulsion contient 0,01 à 25 % en poids de solvants hydrosolubles et/ou liposolubles.

4. Microémulsion selon la revendication 3, **caractérisée en ce que** les solvants sont choisis dans le groupe : alcools monovalents ou polyvalents, polyols, pyrrolidones, dérivés de glucide.

5. Microémulsion selon l'une quelconque des revendications 1 à 4, **caractérisée en ce que** les émulsifiants eau/huile et huile/eau sont non ioniques et les émulsifiants eau/huile présentent une valeur de HLB (hydrophylic-lipophilic balance - équilibre hydrophile-lipophile) de 2 à 7 et les émulsifiants huile/eau présentent une valeur de HLB de 9 à 18.

6. Microémulsion selon la revendication 5, **caractérisée en ce que** les émulsifiants eau/huile et huile/eau sont choisis dans le groupe : alcools gras éthoxylés, dérivés de sorbitane, dérivés de sorbitane éthoxylés, dérivés glycéryliques d'acides gras saturés ou insaturés tels que des dérivés monoglycéryliques, des dérivés diglycéryliques, des dérivés triglycéryliques, des dérivés polyglycéryliques, des esters de glycéryle éthoxylés, des alkyléthers éthoxylés, des glucosides d'alcools gras en C₁₆₋₁₈.

7. Microémulsion selon l'une quelconque des revendications 1 à 6, **caractérisée en ce que** les phospholipides sont choisis dans le groupe : lécithines, phosphatidyléthanolamine, phosphatidylsérine, phosphatidylinositol, lécithine hydroxylée.

8. Microémulsion selon l'une quelconque des revendications 1 à 7, **caractérisée en ce que** la microémulsion contient 1 à 20 % en poids d'un ou plusieurs principes actifs hydrosolubles ou liposolubles.

9. Microémulsion eau/huile secondaire ou microémulsion huile/eau secondaire, constituée d'un mélange d'une microémulsion selon l'une quelconque des revendications 1 à 8 et d'eau ou d'une solution aqueuse en un rapport de 1:1 à 1:100.

10. Microémulsion selon l'une quelconque des revendications 1 à 9 pour une utilisation en tant que médicament.

11. Utilisation non thérapeutique d'une microémulsion selon l'une quelconque des revendications 1 à 10 pour le soin de la peau, pour la régulation de l'humidité de la peau et/ou pour le traitement de signes du vieillissement cutané.
